# EUROPEAN PATENT SPECIFICATION

(11) **EP 4 387 949 B1**
(45) Date of publication and mention of the grant of the patent: **08.10.2025**
(21) Application number: 22772487.9
(22) Date of filing: 31.08.2022
(51) Int. Cl.: C07C 51/38, C07C 59/185

(54) **PROCESS FOR THE PRODUCTION OF LEVULINIC ACID AND DERIVATIVES THEREOF**
VERFAHREN ZUR HERSTELLUNG VON LEVULINSÄURE UND DERIVATEN DAVON
PROCÉDÉ DE PRODUCTION D'ACIDE LÉVULINIQUE ET DÉRIVÉS ASSOCIÉS

(30) Priority: 01.09.2021 EP 21194323
(43) Date of publication of application: 26.06.2024
(73) Proprietor: Universiteit Antwerpen, 2000 Antwerpen (BE)
(72) Inventor: MAES, Bert, 2020 Antwerpen (BE); VER ELST, Céderic, 2020 Antwerpen (BE); BAL, Mathias, 2020 Antwerpen (BE)
(74) Representative: Arnold & Siedsma
(86) International application number: PCT/EP2022/074225
(87) International publication number: WO 2023/031285

(56) References cited:
- WO-A1-2014/189991
- JP-A- 2012 000 059
- US-A1- 2011 282 078
- CARRAHER JACK M. ET AL: "Solvent-driven isomerization of cis , cis -muconic acid for the production of specialty and performance-advantaged cyclic biobased monomers", vol. 22, no. 19, 5 October 2020 (2020-10-05), GB, pages 6444 - 6454, XP055882055, ISSN: 1463-9262, Retrieved from the Internet <URL:https://pubs.rsc.org/en/content/articlepdf/2020/gc/d0gc02108c> DOI: 10.1039/D0GC02108C

## Description

### FIELD OF THE INVENTION

The present invention relates to the field of organic synthesis, and in particular, the present invention pertains to a process for preparing a levulinic acid or salt thereof, or a levulinic acid ester (levulinate).

### BACKGROUND TO THE INVENTION

Levulinic acid (also known as 4-oxopentanoic acid) is a keto acid, therefore bearing two different reactive functional groups: a carbonyl functionality and a carboxyl functionality. The presence of these two groups makes levulinic acid and derivatives thereof versatile intermediates for the synthesis of a large number of derivatives. For example levulinic acid ketals and esters are used as performance solvents and dispersing agents for personal care formulations (anti-aging, anti-acne, skin whitening, sun protection). Levulinate esters are used as flavoring additives in food industry. Further application area is household and industrial cleaners. Industrially relevant compounds which can be manufactured starting from levulinic acid include, among others, levulinate ketals, 5-methyl-2-pyrrolidone, 2-methyltetrahydrofuran (MTHF), succinic acid, γ-valerolactone (GVL), 5-aminolevulinic acid, diphenolic acid (DPA) and levulinate esters.

At present, the production technology and market demand of levulinic acid has not yet developed according to previous expectations and its direct production from biomass is still under development. EP3055283A1 discloses a process for the manufacturing of levulinic acid from sugars. The method described in EP3055283A1, which can be realized on the scale up to 10 thousand tons annually, is from cellulosic biomass or sugar-containing feedstock upon treatment with mineral acids at elevated temperature. The method described in EP3055283A1 comprises a first step of heating a first mixture comprising water and sulfuric acid to about 80 to about 160 °C to form a solution, a second step of adding a second mixture of water and a sugar selected from glucose and sucrose to the heated solution over a first period of time to form a liquid reaction mixture in a reactor, wherein the liquid reaction mixture comprises between 20% and 60% sulfuric acid; and a third step of recovering levulinic acid from the reaction mixture. This process is very sensitive to the reaction parameters such as the temperature regime resulting in strongly varied yields of desired levulinic acid and multiple side products. An important negative feature of this process is the formation of char and a byproduct formic acid (FA). The requirement of separation of the formed formic acid and separation and disposal of char are important drawbacks of the method.

US 2011/282078 discloses processes for preparing cyclohexene-1,4-carboxylates, wherein a portion of the starting materials utilized is derived from renewable resources.

JP 2012 000059 discloses a method for fermentative production of muconolactone, β-ketoadipic acid and/or levulinic acid.

WO 2014/189991 discloses processes to prepare levulinic acid, formic acid and/or hydroxymethyl furfural from various biomass materials.

Therefore, there is an industry need in novel production methods of levulinic acid giving higher yield and requiring less downstream processing then the production methods in the prior art.

### SUMMARY OF THE INVENTION

The present invention aims at overcoming the drawbacks of the prior art. In a first aspect, the present invention relates to a process for preparing a levulinic acid, a salt thereof or levulinate ester, comprising the steps of:
a) providing a reaction mixture, either homogeneous or heterogeneous, comprising:
   - a polar protic solvent, in another words a polar hydrogen bond donor solvent; and
   - a muconic compound selected from the list comprising: muconic acid, muconate salt, muconate ester, mucono(di)lactone (in other words muconolactone or muconodilactone), either substituted or unsubstituted; and
b) heating the reaction mixture provided at step a) at a temperature Tᵢ of at least about 120°C and at a pressure Pᵢ higher or equal to the autogenic pressure, thereby preparing the levulinic acid, levulinate ester or salt thereof.

At step a) the muconic compound is of general formula (I): 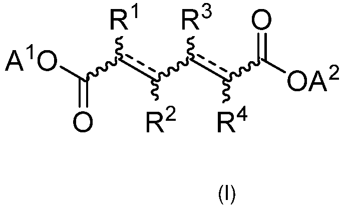 wherein for general formula (I):
- A¹, A² are independently selected from the list comprising: H, alkyl, and a cation;
- R¹, R², R³, R⁴ are independently selected form the list comprising H, alkyl, alkenyl, carboxyl, aryl, and at least one of R², R³ is H;
- R¹C-CR², and R³C-CR⁴ are double C-C bonds;
   or
- A¹ represents a direct bond connecting the oxygen to which it is attached to the carbon atom at the R³ position thereby forming a 5 membered ring;
- A² is selected from the list comprising: H, alkyl, and a cation;
- R¹, R², R⁴ are independently selected from the list comprising: H, alkyl, alkenyl, carboxyl, aryl;
- R¹C-CR² is a double C-C bond, and R³C-CR⁴ is a single C-C bond;
   or
- A¹, respectively A² each represent respectively a direct bond connecting the oxygen to which it is attached to the carbon atom at the R³ position, respectively R² position thereby forming a fused system of two 5-membered rings;
- R¹, R⁴ are independently selected from the list comprising: H, alkyl, alkenyl, carboxyl, aryl;
- R¹C-CR², and R³C-CR⁴ are single C-C bonds.

The levulinic acid, levulinate ester or salt is a compound of formula (II): wherein 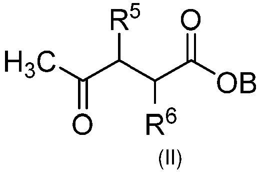
- B selected from the group comprising: H, alkyl, inorganic cation, organic cation;
- R⁵, R⁶ are selected from, and not limited to, the group comprising: H, alkyl, alkenyl, carboxyl, aryl.

The present invention provides for several advantages. An advantage of the present invention is that a levulinic acid, levulinate ester or salt thereof is obtained with higher yields, and by means of less labor intensive methodology compared to prior art. Further, by means of the present invention, the levulinic acid, levulinate ester or salt thereof can be easily separated, as the formation of undesired side-products is reduced. By means of the present invention, only CO₂ is produced as a byproduct, which is easy to separate, as it separates spontaneously. It has been further found that various additives such as inorganic salts such as Na, Li, K, Mg, Ca chlorides, phosphates and sulphates do not significantly influence the high yield of levulinic acid, thereby allowing the reaction to be carried out on non-purified muconic acid i.e. directly from fermentation of bio based feedstock such as sugars. Another advantage of the present invention is that the reaction can be carried out under mild conditions, thereby not requiring the use of corrosion-resistant reactor materials. A further advantage of the present invention is that levulinic acid, levulinate ester or salts thereof can all be obtained by means of a single reaction step, without requiring the need for separation and/or work-up of reaction intermediates.

According to a further embodiment of the present invention, step b) comprises heating the reaction mixture at a temperature Tᵢ of at least 160°C, more preferably at least 180°C. It has been advantageously found that the present embodiment, provides for higher yields, without side reactions to take place.

According to an embodiment of the present invention, at step a) the polar protic solvent is water. An advantage of the present embodiment, is that the reaction is more environmentally friendly whilst providing for high yields.

According to an embodiment of the present invention, at step a) the polar protic solvent is an alcohol. An advantage of the present embodiment, is that the reaction directly provides value-added levulinate esters.

According to an embodiment of the present invention, the reaction mixture further comprises a basic catalyst, in other words a base acting as a catalyst to the reaction. An advantage of the present embodiment, is that the higher yields can be achieved and lower temperature can be used compared to a reaction without such basic catalysts..

According to an embodiment of the present invention, the basic catalyst comprises one or more sp3 hybridized nitrogen atoms, in other words, the catalyst comprises a nitrogen atom having sp3 hybridization.

An advantage of the present embodiment, is that even higher yields can be achieved, and that the base can be easily separated from the levulinate ester product, by means of an extraction.

According to an embodiment of the present invention, the basic catalyst is selected from the list comprising: diisopropylethylamine, 2,2,6,6-tetramethylpiperidine, 1-azabicyclo[2.2.2]octane, 1,4-diazabicyclo[2.2.2]octane, 1,8-bis(dimethylamino)naphthalene. An advantage of the present embodiment, is that these amines provide for the best results.

According to an embodiment of the present invention, step a) comprises providing the muconic compound in a cis,cis-, cis,trans- or trans,cis-configuration, preferably a cis,cis-configuration.

An advantage of starting from the cis,cis-muconic acid is that it is an isomer directly obtainable by fermentation.

According to an embodiment of the present invention, step b) comprises heating the reaction mixture in a non-oxidizing atmosphere, such as N₂, Ar, which is not provided to oxidize the reactants and/or products. An advantage of the present embodiment, is that side reactions negatively affecting the obtainment of products are reduced.

According to an embodiment of the present invention, steps a) and b) are performed subsequently, without isolation of any intermediates. In other words, the process according to the present invention is a one-pot reaction.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings is provided to make apparent to those skilled in the art how the several forms of the invention may be embodied in practice.

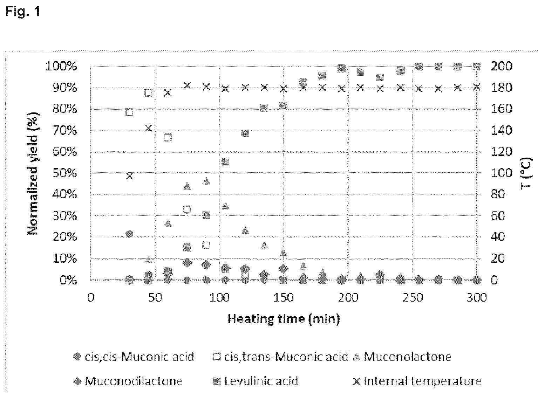

**Figure 1****,** also referred to as **Fig. 1****,** illustrates results of a sampling experiment for the synthesis of levulinic acid from cis,cis-muconic acid, see Example 9.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. When describing the compounds of the invention, the terms used are to be construed in accordance with the following definitions, unless a context dictates otherwise.

The term "about" or "approximately" as used herein when referring to a measurable value such as a parameter, an amount, a temporal duration, and the like, is meant to encompass variations of +/- 10 % or less, preferably +/- 5 % or less, more preferably +/- 1 % or less, and still more preferably +/- 0.1 % or less of and from the specified value, insofar such variations are appropriate to perform in the disclosed invention. It is to be understood that the value to which the modifier "about" or "approximately" refers is itself also specifically, and preferably, disclosed.

In a first aspect, the present invention relates to a process for preparing a levulinic acid, or salt thereof or levulinate ester, comprising the steps of:
a) providing a reaction mixture comprising:
   - a polar protic solvent; and
   - a muconic compound selected from the list comprising: muconic acid, muconate salt, muconate ester, mucono(di)lactone and represented by general formula (I): 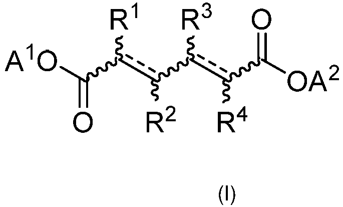 wherein for general formula (I):
      - A¹, A² are independently selected from the list comprising: H, alkyl, and a cation;
      - R¹, R², R³, R⁴ are independently selected form the list comprising H, alkyl, alkenyl, carboxyl, aryl, and at least one of R², R³ is H;
      - R¹C-CR², and R³C-CR⁴ are double C-C bonds;
         or
      - A¹ represents a direct bond connecting the oxygen to which it is attached to the carbon atom at the R³ position thereby forming a 5 membered ring;
      - A² is selected from the list comprising: H, alkyl, and a cation;
      - R¹, R², R⁴ are independently selected from the list comprising: H, alkyl, alkenyl, carboxyl, aryl;
      - R¹C-CR² is a double C-C bond, and R³C-CR⁴ is a single C-C bond;
         or
      - A¹, respectively A² each represent respectively a direct bond connecting the oxygen to which it is attached to the carbon atom at the R³ position, respectively R² position thereby forming a fused system of two 5-membered rings;
      - R¹, R⁴ are independently selected from the list comprising: H, alkyl, alkenyl, carboxyl, aryl;
      - R¹C-CR², and R³C-CR⁴ are single C-C bonds; and
b) heating the reaction mixture provided at step a) at a temperature Ti of at least about 120°C and at a pressure Pi ≥ autogenic pressure, thereby preparing the levulinic acid or salt thereof, or levulinate ester;
   wherein the levulinic acid or salt thereof, or levulinate ester is represented by formula (II): wherein 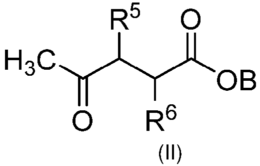
   - B selected from the group comprising: H, alkyl, inorganic cation, organic cation;
   - R⁵, R⁶ are selected from, and not limited to, the group comprising: H, alkyl, alkenyl, carboxyl, aryl.

In particular, levulinic acid corresponds to the compound of formula (II) for which B is H, and R⁵ and R⁶ are H, and a levulinate ester (also referred to in the context of the present invention as simply levulinate or levulinic acid ester) corresponds to the compound of formula (II) for which B is alkyl, whereas a levulinate salt corresponds to the compound of formula (II) for which B is a cation, either organic or inorganic. Levulinic acids, levulinate esters and levulinate salts obtainable according to the present invention can also be substituted, see group R⁵ and R⁶.

The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. C₁-C₆ alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl. The term "optionally substituted alkyl" refers to an alkyl group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

The term "alkenyl", as used herein, unless otherwise indicated, means straight-chain, cyclic, or branched-chain hydrocarbon radicals containing at least one carbon-carbon double bond. Generally, alkenyl groups of this invention comprise from 2 to 20 carbon atoms. Examples of alkenyl radicals include ethenyl, E- and Z-propenyl, isopropenyl, E- and Z-butenyl, E- and Z-isobutenyl, E- and Z-pentenyl, E- and Z-hexenyl, E,E-, E,Z-, Z,E-, Z,Z-hexadienyl, and the like. An optionally substituted alkenyl refers to an alkenyl having optionally one or more substituents (for example 1, 2, 3 or 4), selected from those defined above for substituted alkyl. The term "alkynyl", as used herein, unless otherwise indicated, means straight-chain or branched-chain hydrocarbon radicals containing at least one carbon-carbon triple bond. Examples of alkynyl radicals include ethynyl, propynyl, butynyl, pentynyl, hexynyl, and the like. An optionally substituted alkynyl refers to an alkynyl having optionally one or more substituents (for example 1, 2, 3 or 4), selected from those defined above for substituted alkyl.

The term "cycloalkyl" by itself or as part of another substituent is a cyclic alkyl group, that is to say, a monovalent, saturated, or unsaturated hydrocarbyl group having 1, 2, or 3 cyclic structure. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 15 atoms. The further rings of multi-ring cycloalkyls may be either fused, bridged and/or joined through one or more spiro atoms. Cycloalkyl groups may also be considered to be a subset of homocyclic rings discussed hereinafter. Examples of cycloalkyl groups include but are not limited to cyclopropyl, cyclobutyl, cyclopentyl, cyclohexyl, cycloheptyl, cyclooctyl, cyclononyl, adamantanyl and cyclodecyl with cyclopropyl being particularly preferred. An "optionally substituted cycloalkyl" refers to a cycloalkyl having optionally one or more substituents (for example 1 to 3 substituents, for example 1, 2, 3 or 4 substituents), selected from those defined above for substituted alkyl. When the suffix "ene" is used in conjunction with a cyclic group, hereinafter also referred to as "Cycloalkylene", this is intended to mean the cyclic group as defined herein having two single bonds as points of attachment to other groups. Cycloalkylene groups of this invention preferably comprise the same number of carbon atoms as their cycloalkyl radical counterparts. Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene. Similarly, where alkenyl groups as defined above and alkynyl groups as defined above, respectively, are divalent radicals having single bonds for attachment to two other groups, they are termed "alkenylene" and "alkynylene" respectively.

Generally, alkylene groups of this invention preferably comprise the same number of carbon atoms as their alkyl counterparts. Where an alkylene or cycloalkylene biradical is present, connectivity to the molecular structure of which it forms part may be through a common carbon atom or different carbon atom, preferably a common carbon atom. To illustrate this applying the asterisk nomenclature of this invention, a C₃ alkylene group may be for example *-CH₂CH₂CH₂-*, *-CH(-CH₂CH₃)-*, or *-CH₂CH(-CH₃)-*. Likewise a C₃ cycloalkylene group may be

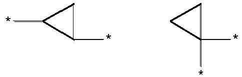

Where a cycloalkylene group is present, this is preferably a C₃-C₆ cycloalkylene group, more preferably a C₃ cycloalkylene (i.e. cyclopropylene group) wherein its connectivity to the structure of which it forms part is through a common carbon atom. Cycloalkylene and alkylene biradicals in compounds of the invention may be, but preferably are not, substituted.

The term "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl. The aryl ring can optionally be substituted by one or more substituents. An "optionally substituted aryl" refers to an aryl having optionally one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO₂-NH₂, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO₂R^{a}, alkylthio, carboxyl, and the like, wherein R^{a} is alkyl or cycloalkyl. Where a carbon atom in an aryl group is replaced with a heteroatom, the resultant ring is referred to herein as a heteroaryl ring.

An "optionally substituted heteroaryl" refers to a heteroaryl having optionally one or more substituents (for example 1 to 4 substituents, for example 1, 2, 3 or 4), selected from those defined above for substituted aryl. The term "oxo" as used herein refers to the group =O.

The term "alkoxy" or "alkyloxy" as used herein refers to a radical having the Formula -OR^{b} wherein R^{b} is alkyl. Preferably, alkoxy is C₁-C₁₀ alkoxy, C₁-C₆ alkoxy, or C₁-C₄ alkoxy. Non-limiting examples of suitable alkoxy include methoxy, ethoxy, propoxy, isopropoxy, butoxy, isobutoxy, sec-butoxy, tert-butoxy, pentyloxy and hexyloxy. Where the oxygen atom in an alkoxy group is substituted with sulfur, the resultant radical is referred to as thioalkoxy.

The term "aryloxy" as used herein denotes a group -O-aryl, wherein aryl is as defined above.

The term "arylcarbonyl" or "aroyl" as used herein denotes a group -C(O)-aryl, wherein aryl is as defined above. The term "carboxy" or "carboxyl" or "hydroxycarbonyl" by itself or as part of another substituent refers to the group -CO₂H. Thus, a carboxyalkyl is an alkyl group as defined above having at least one substituent that is -CO₂H. The term "alkoxycarbonyl" by itself or as part of another substituent refers to a carboxy group linked to an alkyl radical i.e. to form - C(=O)OR^{e}, wherein R^{e} is as defined above for alkyl. The term "alkylcarbonyloxy" by itself or as part of another substituent refers to a -O-C(=O)R^{e} wherein R^{e} is as defined above for alkyl. The term "alkoxy" by itself or as part of another substituent refers to a group consisting of an oxygen atom attached to one optionally substituted straight or branched alkyl group, cycloalkyl group, aralkyl, or cycloalkylalkyl group. Non-limiting examples of suitable alkoxy group include methoxy, ethoxy, n-propoxy, isopropoxy, n-butoxy, isobutoxy, sec-butoxy, tert-butoxy, hexanoxy, and the like. The term "halo" or "halogen" as a group or part of a group is generic for fluoro, chloro, bromo, or iodo. Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound.

The present invention comprises a first step a) of providing a reaction mixture comprising a polar protic solvent; and a muconic compound.

In the context of the present invention, by means of the term "reaction mixture", reference is made to a mixture, either homogeneous or heterogeneous, adapted to be heated under pressure in e.g. a pressurized reactor. For example, the reaction mixture according to the present application is a solution and/or suspension of one or more compounds provided to be heated under pressure in accordance with the present application. Therefore, in accordance with the present application, the muconic compound can either be completely dissolved, partially dissolved or undissolved (thereby providing a suspension) in a polar protic solvent. In the context of the present invention, it has to be understood that other than a polar protic solvent, the reaction mixture can comprise one or more other solvents which are not protic. For example, the reaction mixture can comprise a solution of methanol and acetone or water and acetone in various proportions.

In a specific embodiment according to the present invention, the reaction mixture further comprises one or more additives. Therefore, one or more additives can be added to the reaction mixture so to fine-tune the reaction conditions and e.g. improve yields. It has been found that the process according the present invention is compatible with the use of either acidic or basic additives.

In the context of the present invention, by means of the term "polar protic solvent", reference is made to one or more of liquid compounds both polar and protic. Liquid compounds providing for both polar and protic properties are for example those comprising one or more highly electronegative atoms such as O, N, F, bound to a hydrogen atom. Examples of polar protic solvents are, but not limited to, acetic acid, n-butanol, isopropanol, n-propanol, ethanol, methanol, formic acid and water.

According to an embodiment of the present invention, at step a) the polar protic solvent is selected from the list comprising: water, polar protic organic solvent. Examples of polar protic organic solvents are, but not limited to, acetic acid, n-butanol, isopropanol, n-propanol, ethanol, methanol, formic acid. By means of the term "organic solvent" reference is made to a solvent which is carbon based, in other words, that it contains at least one, or more, carbon atoms.

According to an embodiment of the present invention, at step a) the polar protic solvent is selected from the list comprising: water, alkyl alcohol e.g. methanol, ethanol, and mixtures thereof. According to an embodiment of the present invention, at step a) the polar protic solvent is water. The use of water as polar protic solvent is advantageous for several reasons, in particular, water is a non-toxic, non-flammable, readily available and very cheap solvent which does not provide inconveniences such as its waste disposal, as it can be separated from the obtained levulinic compounds obtained simply by evaporation. Further, its stability and it being non-flammable renders water a better solvent to work with overall, especially in an industrial setting. In accordance with the present application, water can be also used without the addition of any acid and or base thereto. This further simplifies the implementation of the process according to the present invention, avoiding the use of corrosive bases and acids. Therefore, in accordance with a further embodiment, the process of the present invention can be carried out in the absence of an acid and/or base dissolved in water. The use of water as a polar protic solvent enables the production of levulinic acid from a variety of substrates, providing high yields. In particular, the use of water as a polar protic solvent provides levulinic acid from all of cis,cis-muconic acid, cis,trans-muconic acid, muconolactone and muconodilactone (muconic acid dilactone), even without the use of additives, e.g. bases or acids. This enables a flexible synthetic strategy adapted to act on a variety of interrelated substrates.

Therefore, in accordance with an embodiment of the present invention, at step a) water is not provided to contain any basic or acid additives, so that the reaction mixture is not provided with an adjusted pH, which differs from the pH of the reaction mixture provided by the muconic compound or compounds alone. In accordance with a further specific embodiment of the present invention, at step a) the reaction mixture consists of water and one or more muconic compounds. In yet a further specific embodiment of the present invention, at step a) the reaction mixture consists of a polar protic organic solvent and one or more muconic compounds. In yet a further specific embodiment of the present invention, at step a) the reaction mixture consists of an alkyl alcohol and one or more muconic compounds.

In case levulinic acids need to be obtained from trans,trans-muconic acid, water can also be used as a solvent. It has been found that the use of an additive, e.g. an acid additive (such as HCl), or by increasing the reaction temperature, e.g. to 250 °C, is beneficial to this process.

According to an embodiment of the present invention, at step a) the polar protic solvent is an alkyl alcohol. An advantage of the present embodiment is that levulinic acid alkyl esters, in other words, alkyl levulinates, can be obtained in a one-pot approach, without the need for performing alkylation on an obtained levulinic acid in a subsequent step, thereby saving in synthetic resources. According to yet a further embodiment, the polar protic solvent is a C₁₋₄alkyl alcohol, more in particular selected from the group comprising: methanol, ethanol, isopropanol, propanol and butanol. According to yet a further embodiment of the present invention, at step a) the polar protic solvent is methanol.

In the context of the present invention, by means of the term "muconic compound", reference is made to an unsaturated 1,6-dicarboxylic compound, or (di)lactone thereof, such as muconic acid, muconic salt (organic or inorganic salt of the muconic acid, also known as muconate salts), muconic ester (also known as muconates), having either cis,cis-, trans,trans-, cis,trans- and trans,cis-configuration, muconolactone or muconodilactone. Muconic compounds can be obtained by means of methodologies in the state of the art, such as from the oxidation of catechols or fermentation of carbohydrates.

In accordance with the present application, at step a) the muconic compound is of general formula (I):

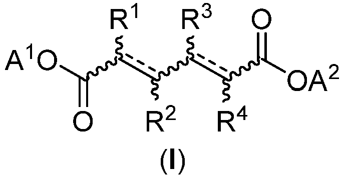

In general formula (I), wavy bonds denote the possibility for the atoms participating to the bond of being in cis,cis-, trans,trans-, cis,trans- or trans,cis-configuration. By means of cis,cis-, trans,trans-, cis,trans- or trans,cis-configuration, reference is made to the following (exemplifying, without limiting, with unsubstituted muconic acid):

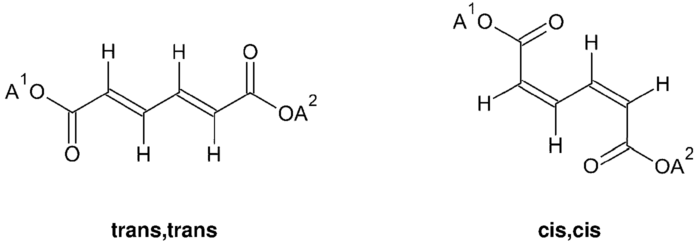

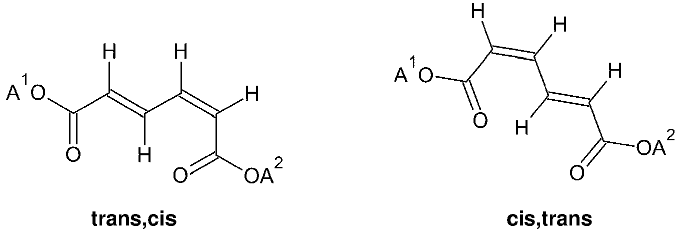

According to an embodiment of the present invention, step a) comprises providing the muconic compound in a cis,cis-, cis,trans- or trans,cis- configuration, preferably a cis,cis-configuration. Cis,cis-configuration is preferred because these isomers are more readily available. For example, the cis,cis-muconic acid isomer is can be obtained by fermentation of sugars. This makes the cis,cis-isomer easier to obtain. According to a specific embodiment of the present invention, step a) comprises providing muconic acid, ester or salt thereof in a cis,cis-, cis,trans- or trans,cis- configuration, preferably a cis,cis-configuration.

By means of the process according to the present invention, different substituents at position R¹, R², R³ and R⁴ are well tolerated, thereby enabling a convenient synthetic route to substituted levulinic acids, esters and salts thereof.

Further, in general formula (I) the C-C bonds in R¹C-CR² and R³C-CR⁴ can either be single bonds or double bonds. This is denoted by the use of a single partially dotted bond in general formula (I).

General formula (I) above therefore denotes stereoisomers (cis,cis, cis,trans etc.) and structural isomers ((di)lactones) of muconic acid, and esters and salts thereof. In particular, various group formulas are encompassed by general formula (I). General formula (I) can either be defined as:
- A¹, A² are independently selected from the list comprising: H, alkyl, and a cation;
- R¹, R², R³, R⁴ are independently selected form the list comprising H, alkyl, alkenyl, carboxyl, aryl, and at least one of R², R³ is H;
- R¹C-CR², and R³C-CR⁴ are double C-C bonds;
thereby constituting group formula (IA);

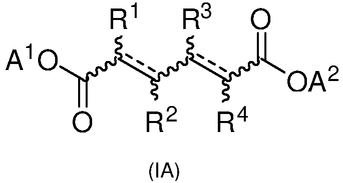

Group formula (IA) therefore denotes cis,cis-, cis,trans- or trans,cis-, trans,trans-muconic acids, esters and salts thereof. The compounds referred to herein can further be provided with various substituents at any one of positions R¹, R², R³ and R⁴, either identical or different, nevertheless, at least one of R², R³ is H. Compounds referred herein can also be provided unsubstituted (R¹ = R² = R³ = R⁴ = H). In accordance with a specific embodiment of the present invention, the muconic compound is of group formula (IA).

Otherwise, general formula (I) can be defined as:
- A¹ represents a direct bond connecting the oxygen to which it is attached to the carbon atom at the R³ position thereby forming a 5 membered ring;
- A² is selected from the list comprising: H, alkyl, and cation;
- R¹, R² and R⁴ are independently selected from the list comprising: H, alkyl, alkenyl, carboxyl, aryl;
- R¹C-CR² is a double C-C bond, and R³C-CR⁴ is a single C-C bond;
thereby constituting formula group (IB);

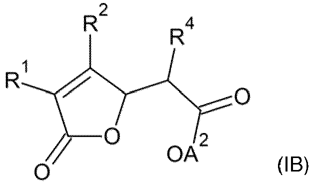

Group formula (IB) therefore denotes a monolactone of muconic acid (also referred to as muconolactone), esters and salts thereof, either substituted at any one of positions R¹, R² and/or R⁴, or unsubstituted (R¹ = R² = R⁴ = H). R¹, R² and R⁴ can differ. In accordance with a specific embodiment of the present invention, the muconic compound is of group formula (IB). In other words, in accordance with a specific embodiment of the present invention, the muconic compound is a monolactone of muconic acid, or an ester or a salt thereof.

Otherwise, general formula (I) can be defined as:
- A¹, respectively A² each represent respectively a direct bond connecting the oxygen to which it is attached to the carbon atom at the R³ position, respectively R² thereby forming a fused system of two 5-membered rings;
- R¹, R⁴ are independently selected from the list comprising: H, alkyl, alkenyl, carboxyl, aryl;
- R¹C-CR², and R³C-CR⁴ are single C-C bonds;
thereby constituting formula group (IC);

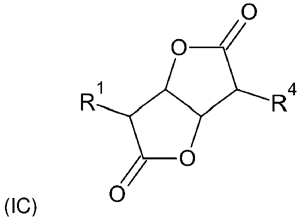

Group formula (IC) therefore denotes a dilactone of muconic acid which can either substituted at any one of positions R¹ and/or R⁴, or unsubstituted (R¹ = R⁴ = H). When unsubstituted, the group formula (IC) represents muconodilactone. In accordance with a specific embodiment of the present invention, the muconic compound is muconodilactone.

The present invention further comprises the step:
b) heating the reaction mixture provided at step a) at a temperature Tᵢ of at least about 120°C and at a pressure Pᵢ ≥ autogenic pressure, thereby preparing the levulinic acid or salt thereof, or levulinate.

According to the present step, the reaction mixture provided at step a), which comprises a polar protic solvent and a muconic compound according to formula (I), is heated at a temperature of at least 120°C under pressure, which is at least autogenic pressure. It has to be understood that the autogenic pressure is dependent on the temperature of the reaction mixture, the solvent and the headspace (hence the reactor design). For example, the theoretical pressures for water are 2 bara at 120 °C, 10 bara at 180 °C, and 16 bara at 200 °C.

In the context of the present invention, by means of the term "autogenic pressure" or "autogenous pressure", reference is made to the pressure self-generated inside a closed container. The pressure is required for preventing the polar protic solvent from boiling.

According to a further embodiment of the present invention, step b) comprises heating the reaction mixture at a temperature Tᵢ of at least 140°C, preferably at least 160°C, more preferably at least 180°C. It has been found that higher temperatures are beneficial and in particular higher yields can be obtained. Good yields can be already obtained at a temperature of at least 160°C. At a temperature of above 180°C, quantitative yields can be obtained.

In the context of the present invention, by means of the term "alkyl alcohol", reference is made to an alcohol of general formula R(OH)n, wherein R is an alkyl, and n corresponds to the number of hydroxyl groups, e.g. for n=2 the alkyl alcohol is a diol, for n=3 the alkyl alcohol is a triol and so forth.

According to an embodiment of the present invention, the polar protic solvent at step a) comprises an additive selected from the list comprising: acids and bases. Possible acids which could be used as additives according to the present invention can be organic acids, such as formic, acetic, or propionic, or mineral acids, such as HCl or H₂SO₄. In the context of the present invention, by means of the term "mineral acid", reference is made to an acid derived from one or more inorganic compounds, such as HCl, HBr, H₂SO₄, H₃PO₄. Further, possible bases which could be used as additives according to the present invention include metal hydroxides such as NaOH or KOH. In the context of the present invention, by means of the term "metal hydroxide", reference is made to a base comprising at least one hydroxide anion and a metallic cation, such as NaOH, KOH, LiOH, Ca(OH)₂. It has been found that the use of an acid, preferably a mineral acid, and a base, preferably a metal hydroxide, can be beneficial in providing higher yields, depending on the substrate used. The use of additives, either acids or bases, is not required by the present invention. In other words, it should be clear that levulinic acids, salts or ester thereof can be obtained in accordance with the present application without the presence of any acid or base.

According to an embodiment of the present invention, the reaction mixture further comprises a basic catalyst. In the context of the present invention, by means of the term "basic catalyst", reference is made to a base which is provided to catalyze a reaction. Examples of basic catalysts in accordance with the present application are, but not limited to, carboxylate salts, such as and not limited to, sodium acetate, ammonium acetate, metal hydroxides, such as, and not limited to, aluminum hydroxide, compounds comprising sp3 nitrogen, such as, and not limited to, ammonia, and compound comprising sp2 hybridized nitrogen atom, such as, and not limited to, pyridine. According to a preferred embodiment of the present invention, the basic catalyst comprises at least one sp3 hybridized nitrogen atom.

According to an embodiment of the present invention, the basic catalyst is selected from the list comprising: diisopropylethylamine, 2,2,6,6-tetramethylpiperidine, 1-azabicyclo[2.2.2]octane, 1,4-diazabicyclo[2.2.2]octane, 1,8-bis(dimethylamino)naphthalene. It has been found that the present basic catalysts are capable of providing for the highest yields.

According to an embodiment of the present invention, at step a) the muconic compound is of group formula (IA), wherein A¹ and A² are H, and either
- the additive is a mineral acid, preferably at a concentration of at least 1 eq., preferably 2 eq., preferably 5 eq., preferably 10 eq., or
- the additive is a metal hydroxide, preferably at a concentration in a range from about 0.1 eq., to about 1.5 eq., preferably 1 eq.

According to an embodiment of the present invention, at step a) the muconic compound is of group formula (IA), wherein A¹ and A² are H, and the polar protic solvent is an alkyl alcohol.

According to an embodiment of the present invention, step b) comprises heating the reaction mixture in a non-oxidizing atmosphere. In the context of the present invention, by means of the term "non-oxidizing atmosphere", reference is made to an atmosphere which comprises one or more gases which are provided to not oxidize any one of the reagents, products or intermediates of the process according to the present invention.

In accordance with an embodiment of the present invention, step b) comprises heating the reaction mixture in an atmosphere of a gas selected from the list comprising: H₂, N₂, CO and CO₂. It has been found beneficial to carry out the reaction in one of these gases, or mixtures thereof.

According to an embodiment of the present invention, steps a) and b) are performed subsequently, without isolation of any intermediates. This is advantageous in that it allows for a one-pot reaction saving both time and synthetic steps.

### EXPERIMENTAL PART

### General Procedure A

A 4 mL glass vial was charged with a magnetic stirring bar, the substrate for the experiment, additives (if required) and 3 mL of the appropriate solvent or solvent mixture. The vial was closed properly with the correct cap and septum and the septum was pierced with a syringe needle. This vial was placed in a home-made heating block in the 4596 Parr reactor (25 mL capacity) and the reactor was closed properly. The reactor was flushed with the appropriate gas (3x 10 bar) and then pressurized with the reported pressure of this gas. The reactor was heated to the indicated reaction temperature. The reactor was maintained at this temperature for the indicated reaction time and then cooled down in an ice bath. The gas was released and the reactor was opened. After opening the reactor, a weighed amount of internal standard was added to the crude reaction mixture, along with acetone until the glass vial was full. After stirring well, the mixture was brought into a pear-shaped flask and the glass vial rinsed with water (about 1 mL) and acetone (about 1 mL). If the reaction took place in a non-aqueous solvent, the vial was rinsed with acetone (2x about 1 mL) instead. About half of the combined mixture was taken and evaporated. (If the reaction took place in a non-aqueous solvent, the entire mixture was evaporated and the evaporation was done at a pressure above 60 mbar.) The residue was dissolved in a suitable deuterated solvent and analyzed with NMR.

### General Procedure B

A home-made PTFE liner was charged with the substrate for the experiment, additives (if required) and 9 mL of the appropriate solvent or solvent mixture. This insert was placed in the 4596 Parr reactor (25 mL capacity) equipped with a mechanical stirrer and the reactor was closed properly. The reactor was flushed with the appropriate gas (3x 10 bar) and then pressurized with the reported pressure of this gas. The reactor was heated to the indicated reaction temperature. The reactor was maintained at this temperature for the indicated reaction time and then cooled down in an ice bath. The gas was released and the reactor was opened. After opening the reactor, a weighed amount of internal standard was added to the crude reaction mixture, along with acetone until the insert was full. After stirring well, the mixture was brought into a pear-shaped flask and the vial rinsed with water (about 1 mL) and acetone (about 1 mL). About half of the combined mixture was taken and evaporated. The residue was dissolved in a suitable deuterated solvent and analyzed with NMR.

### General Procedure C

A 4 mL glass vial was charged with a magnetic stirring bar, the substrate for the experiment, additives (if required) and 3 mL of the appropriate solvent or solvent mixture. The vial was closed properly with the correct cap and septum and the septum was pierced with a syringe needle. This vial was placed in a home-made heating block in the 4651 Parr reactor (300 mL capacity) and the reactor was closed properly. The reactor was flushed with the appropriate gas (3x 10 bar) and then pressurized with the reported pressure of this gas. The reactor was heated to the indicated reaction temperature in 1 h. The reactor was maintained at this temperature for the indicated reaction time and then cooled down (in the air until 180 °C was reached, then in an ice bath to RT). The gas was released and the reactor was opened. Unless mentioned otherwise, after opening the reactor, a weighed amount of internal standard was added to the crude reaction mixture, along with acetone until the glass vial was full. After stirring well, the mixture was brought into a pear-shaped flask and the glass vial rinsed with water (about 1 mL) and acetone (about 1 mL). If the reaction took place in a non-aqueous solvent, the vial was rinsed with acetone (2x about 1 mL) instead. About half of the combined mixture was taken and evaporated. (If the reaction took place in a non-aqueous solvent, the entire mixture was evaporated and the evaporation was done at a pressure above 60 mbar.) The residue was dissolved in a suitable deuterated solvent and analyzed with NMR.

### EXAMPLES

### Example 1: Temperature screening for the synthesis of levulinic acid from cis,cis-muconic acid

These experiments were performed according to General Procedure A, using *cis,cis-*muconic acid (43 mg, 0.3 mmol) and water (3 mL) for 3 h under 50 bar of initial N₂ pressure. The ¹H NMR yields (dimethyl sulfone or dimethylmalonic acid internal standard) are reported in the table below.

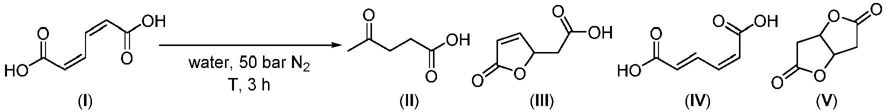

| Experiment #^{c} | T (°C) | Recovered I (%)^{a} | Yield II (%)^{a} | Yield III (%)^{a} | Yield IV (%)^{a} | Yield V (%)^{a} |
|---|---|---|---|---|---|---|
| 1 | 40 | 89 | 0 | 0 | 20 | 0 |
| 2 | 60 | 13 | 0 | 7 | 88 | 0 |
| 3 | 80 | 1 | 0 | 14 | 78 | 0 |
| 3bis | 80 | 0 | 0 | 14 | 84 | 0 |
| 4 | 100 | 1 | 0 | 37 | 56 | 3 |
| 5 | 110 | 0 | trace | 54 | 38 | 8 |
| 6 | 120 | 0 | 9 | 66 | 10 | 14 |
| 7 | 140 | 0 | 24 | 66 | 0 | 16 |
| 8 | 160 | 0 | 61 | 32 | 0 | 7 |
| 9 | 180 | 0 | 92 | 3 | 0 | 0 |
| 10 | 190 | 0 | 103 | 5 | 0 | 0 |
| 11 | 200 | 0 | 102 | 0 | 0 | 0 |
| 12 | 250 | 0 | 95 | 0 | 0 | 0 |
| 13 | 300 | 0 | 98^{b} | 0 | 0 | 0 |

| | | | | | | |
|---|---|---|---|---|---|---|
| ^{a} As there is an experimental error on each individual value determined by ¹H NMR, the mass balance can be more than 100%. ^{b} Reaction under 90 bar of N₂ pressure, impurities were visible in the ¹H NMR spectrum. ^{c} The experiment containing "bis" in the experiment number refers to a duplicate of the experiment with the same number, performed using a different internal standard. Similar yields were obtained as in the original experiment. | | | | | | |

### Example 2: Pressure screening for the synthesis of levulinic acid from cis,cis-muconic acid

These experiments were performed according to General Procedure B, using *cis,cis-*muconic acid (128 mg, 0.9 mmol) and water (9 mL) for 3 h at 180 °C under the indicated N₂ pressure. The ¹H NMR yields (dimethyl sulfone internal standard) are reported in the table below.

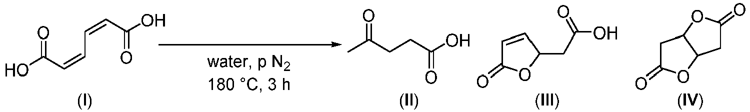

| Experiment # | p (bar) | Yield II (%)^{a} | Yield III (%)^{a} | Yield IV (%)^{a} |
|---|---|---|---|---|
| 14 | 5 | 84 | 13 | 3 |
| 15 | 10 | 88 | 11 | 4 |
| 16 | 15 | 84 | 13 | 4 |
| 17 | 30 | 88 | 10 | 2 |
| 18 | 50 | 83 | 11 | 4 |
| 19 | 75 | 83 | 10 | 2 |
| 20 | 100 | 89 | 13 | 5 |

| | | | | |
|---|---|---|---|---|
| ^{a} As there is an experimental error on each individual value determined by ¹H NMR, the mass balance can be more than 100%. | | | | |

### Example 3: Synthesis of levulinic acid from cis,cis-muconic acid under autogenic pressure [Experiment # 21]

A 10 mL glass vial equipped with a magnetic stirring bar was charged with *cis,cis-*muconic acid (43 mg, 0.3 mmol) and water (3 mL). The vial was sealed with a crimp cap and a metal screw cap, and placed in a preheated oil bath at 180 °C for 3 h. After cooling down to RT, the vial was opened and a weighed amount of internal standard (dimethyl sulfone) was added to the reaction mixture, along with acetone (about 1 mL). When all solids had dissolved, the mixture was transferred to a pear-shaped flask and the vial washed with water (about 1 mL) and acetone (about 1 mL). About 3 mL of the mixture was then evaporated and the residue analyzed with quantitative NMR. Levulinic acid was obtained in 83% ¹H NMR yield, along with muconolactone (2,5-dihydro-5-oxo-2-furanacetic acid, 21% ¹H NMR yield) and muconic acid dilactone (tetrahydrofuro[3,2-b]furan-2,5-dione, 5% ¹H NMR yield).

As there is an experimental error on each individual value determined by ¹H NMR, the mass balance can be more than 100%.

### Example 4: Atmosphere screening for the synthesis of levulinic acid from cis,cis-muconic acid

These experiments were performed according to General Procedure C, using *cis,cis-*muconic acid (43 mg, 0.3 mmol) and water (3 mL) for 3 h at 180 °C under 15 bar of pressure of the indicated gas. The ¹H NMR yields (dimethyl sulfone internal standard) are reported in the table below. (Each experiment was performed in triplicate and the average yields are reported with their standard deviations.)

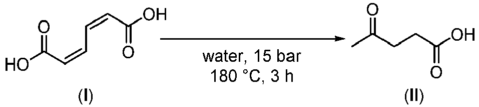

| Experiment # | Atmosphere | Yield II (%)^{a} |
|---|---|---|
| 22 | N2 | 99 ±5 |
| 23 | air | 65 ±3^{b} |
| 24 | H2 | 105 ±4 |
| 25 | CO₂ | 93 ±4 |
| 26 | CO | 103 ±4 |

| | | |
|---|---|---|
| ^{a} As there is an experimental error on each individual value determined by ¹H NMR, the mass balance can be more than 100%. ^{b} A brown reaction mixture was obtained. | | |

### Example 5: Concentration screening for the synthesis of levulinic acid from cis,cis-muconic acid

These experiments were performed according to General Procedure C, using *cis,cis-*muconic acid and water (2-3 mL) for 3 h at 180 °C under 15 bar N₂ pressure. The ¹H NMR yields (dimethyl sulfone internal standard) are reported in the table below.

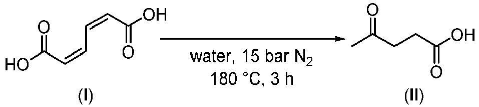

| Experiment # | c (M) | Yield II (%)^{a} |
|---|---|---|
| 27 | 0.10 | 102 |
| 28 | 0.25 | 98 |
| 29 | 0.50 | 96 |
| 30 | 1.0 | 90 |
| 31 | 1.5 | 93 |
| 32 | 2.0 | 91 |
| 33 | 3.0 | 92 |

| | | |
|---|---|---|
| ^{a} As there is an experimental error on each individual value determined by ¹H NMR, the mass balance can be more than 100%. | | |

### Example 6: Additive screening for the synthesis of levulinic acid from cis,cis-muconic acid

These experiments were performed according to General Procedure C, using *cis,cis-*muconic acid (43 mg, 0.3 mmol), the indicated additive (0.6 mmol, 2 eq.) and water (3 mL) for 3 h at 180 °C under 15 bar N₂ pressure. The ¹H NMR yields (dimethyl sulfone internal standard) are reported in the table below.

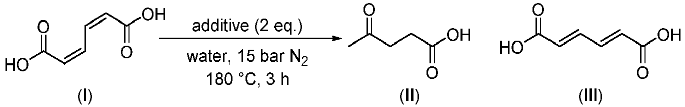

| Experiment # | Additive | II (%)^{a} | III (%)^{a} | Experiment #^{c} | Additive | II (%)^{a} | III (%)^{a} |
|---|---|---|---|---|---|---|---|
| 34 | LiCl | 102 | 0 | 34bis | LiCl | 92 | 0 |
| 35 | NaCl | 103 | 0 | | | | |
| 36 | KCI | 110 | 0 | 36bis | KCl | 94 | 0 |
| 37 | MgCl₂ | 103 | 0 | 37bis | MgCl₂ | 100 | 0 |
| 38 | CaCl₂ | 97 | 0 | 38bis | CaCl₂ | 104 | 0 |
| 39 | NH₄Cl | 99 | 0 | 39bis | NH₄Cl | 108 | 0 |
| 40 | LaCl₃ | 107 | 0 | 40bis | LaCl₃ | 101 | 0 |
| 41 | LiBr | 103 | trace | 41bis | LiBr | 96 | 1 |
| 42 | NaBr | 101 | 1 | 42bis | NaBr | 95 | 1 |
| 43 | KBr | 101 | 3 | 43bis | KBr | 98 | 1 |
| 44 | MgBr₂ | 99 | trace | 44bis | MgBr₂ | 100 | 1 |
| 45 | NH₄Br | 98 | 1 | 45bis | NH₄Br | 103 | trace |
| 46 | Na₂SO₄ | 102 | 0 | 46bis | Na₂SO₄ | 110 | 0 |
| 47 | MgSO₄ | 106 | 0 | 47bis | MgSO₄ | 103 | 0 |
| 48 | CuSO₄ | 75^{b} | 0 | | | | |
| 49 | FeSO₄ | 105 | 0 | | | | |
| 50 | KH₂PO₄ | 102 | 0 | | | | |

| | | | | | | | |
|---|---|---|---|---|---|---|---|
| ^{a} As there is an experimental error on each individual value determined by ¹H NMR, the mass balance can be more than 100%. ^{b} A significant amount of char was formed. ^{c} Experiments containing "bis" in the number refer to a duplicate of the experiment with the same experiment number, after recalibration of the temperature controller of the reactor. Similar yields were obtained as in the original experiments. | | | | | | | |

### Example 7: Acid concentration screening for the synthesis of levulinic acid from cis,cis-muconic acid

These experiments were performed according to General Procedure C, using *cis,cis-*muconic acid (43 mg, 0.3 mmol), the indicated equivalents of HCl and water (3 mL) for 3 h at 180 °C under 15 bar N₂ pressure. The ¹H NMR yields (dimethyl sulfone internal standard) are reported in the table below.

In order to measure the pH at the corresponding concentration of HCl and *cis,cis*-muconic acid, to 20 mL glass vials was added *cis,cis*-muconic acid (1.0 mmol) and water (10 mL), along with the indicated equivalents of HCl. After mixing well, the mixtures were refluxed with a heat gun until all the contents were fully dissolved. (In case this caused significant solvent loss, additional water was added to the mixtures until the solution had a volume of approximately 10 mL.) The pH of the solutions was then measured at RT.

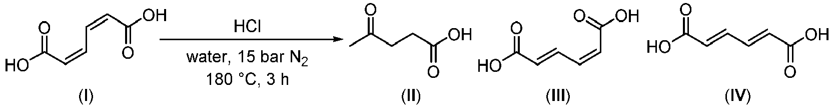

| Experiment #^{c} | eq. HCl | | | c_{HCl} (M) | | | | pH^{a} | | | | Yield II (%)^{b} | | | | Yield III (%)^{b} | Yield IV (%)^{b} |
|---|---|---|---|---|---|---|---|---|---|---|---|---|---|---|---|---|---|
| 22A | 0.0 | | | 0.00 | | | | 1.54 | | | | 99 | | | | 0 | 0 |
| 51 | 1.0 | | | 0.10 | | | | 0.52 | | | | 97 | | | | 0 | 0 |
| 52 | 2.0 | | | 0.20 | | | | 0.32 | | | | 93 | | | | 0 | 0 |
| 52bis | 2.0 | 0.20 | | 0.32 | 104 | | | 0 | | | 0 | | | | | | |
| 53 | 5.0 | | | 0.50 | | | | < 0 | | | | 96 | | | | 0 | 0 |
| 53bis | 5.0 | 0.50 | | < 0 | 104 | | 0 | | | 0 | | | | | | | |
| 54 | 10 | | | 1.00 | | | | < 0 | | | | 98 | | | | 0 | 0 |
| 54bis | 10 | 1.00 | < 0 | 95 | | 0 | | | 0 | | | | | | | | |

| | | | | | | | | | | | | | | | | | |
|---|---|---|---|---|---|---|---|---|---|---|---|---|---|---|---|---|---|
| ^{a} Measured at room temperature. ^{b} As there is an experimental error on each individual value determined by ¹H NMR, the mass balance can be more than 100%. ^{c} Experiments containing "bis" in the number refer to a duplicate of the experiment with the same experiment number, after recalibration of the temperature controller of the reactor. Similar yields were obtained as in the original experiments. | | | | | | | | | | | | | | | | | |

### Example 8: Base concentration screening for the synthesis of levulinic acid from cis,cis-muconic acid

These experiments were performed according to a modified General Procedure C, using cis,cis-muconic acid (43 mg, 0.3 mmol), the indicated equivalents of NaOH and water (3 mL) for 3 h at 180 °C under 15 bar N₂ pressure. Workup consisted of the addition of internal standard, acetone and concentrated HCl to acidify the solutions. After centrifugation, about half of the mixture was evaporated, dissolved in DMSO-*d₆*, filtered if required and analyzed using NMR. The ¹H NMR yields (dimethyl sulfone internal standard) are reported in the table below.

In order to measure the pH at the corresponding concentration of NaOH and *cis,cis*-muconic acid, to 20 mL glass vials was added *cis,cis*-muconic acid (1.0 mmol) and water (10 mL), along with the indicated equivalents of NaOH. After mixing well, the mixtures were refluxed with a heat gun until all the contents were fully dissolved. (In case this caused significant solvent loss, additional water was added to the mixtures until the solution had a volume of approximately 10 mL.) The pH of the solutions was then measured at RT.

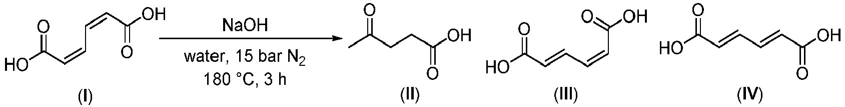

| Experiment #^{c} | eq. NaOH | C_{NaOH} (M) | pH^{a} | Recovered I (%)^{b} | Yield II (%)^{b} | Yield III (%)^{b} | Yield IV (%)^{b} |
|---|---|---|---|---|---|---|---|
| 55 | 0.0 | 0.00 | 1.54 | 0 | 94 | 0 | 0 |
| 56 | 1.0 | 0.10 | 3.51 | 0 | 104 | 0 | 0 |
| 56bis | 1.0 | 0.10 | 3.51 | 0 | 110 | 0 | 0 |
| 57 | 2.0 | 0.20 | 6.18 | trace | 88 | 13 | 2 |
| 58 | 5.0 | 0.50 | 13.05 | 1 | 84 | 11 | 11 |
| 59 | 10 | 1.00 | 13.47 | 1 | 85 | 9 | 10 |
| 59bis | 10 | 1.00 | 13.47 | 1 | 84 | 7 | 14 |

| | | | | | | | |
|---|---|---|---|---|---|---|---|
| ^{a} Measured at room temperature. ^{b} As there is an experimental error on each individual value determined by ¹H NMR, the mass balance can be more than 100%. ^{c} Experiments containing "bis" in the number refer to a duplicate of the experiment with the same experiment number, after recalibration of the temperature controller of the reactor. Similar yields were obtained as in the original experiments. | | | | | | | |

### Example 9: Sampling experiment for the synthesis of levulinic acid from cis,cis-muconic acid [Experiment # 60]

A glass liner was charged with a magnetic stirring bar, *cis,cis*-muconic acid (2.13 g, 15.0 mmol) and water (150 mL). The liner was placed in the 4651 Parr reactor equipped with a dip-tube and sampling valves and the reactor was closed properly. The reactor was flushed with N₂ (3x 10 bar) and then pressurized with N₂ (15 bar). The reactor was heated to the reaction temperature in 1 h and samples (approx. 2.4 mL) were taken every 15 min.

Each sample was degassed by sonication for a few seconds. Then, an accurately measured volume of sample was mixed with an accurately measured volume of stock solution of internal standard (dimethyl sulfone) in acetone. The mixture was evaporated and analyzed using NMR. The ¹H NMR yields of all products present in the sample were normalized to 100% mass balance and plotted in **Fig. 1****.**

**Fig. 1** shows that within the first 30 min of heating, most of the *cis,cis*-muconic acid in the reaction mixture had already isomerized to *cis,trans*-muconic acid. Levulinic acid was detected after 60 min of heating. Muconolactone was formed before Levulinic acid appeared. A small amount of Muconodilactone was also present after 60 min which increased and then disappeared after a longer reaction time. The yield of levulinic acid steadily increased throughout the reaction time, reaching a maximum after 255 min (4 h 15 min) since the start of the heating. This demonstrates that after the reactor reaches its set temperature in one hour, three hours and fifteen minutes are required to reach full conversion.

### Example 10: Synthesis of levulinic acid from cis,cis-muconic acid [Experiment # 61]

A 20 mL glass vial was charged with a magnetic stirring bar, cis,cis-muconic acid (213 mg, 1.50 mmol) and water (15 mL). The vial was closed properly with the correct cap and septum and the septum was pierced with a syringe needle. This vial was placed in a home-made heating block in the 4651 Parr reactor (300 mL capacity) and the reactor was closed properly. The reactor was flushed with the nitrogen (3x 10 bar) and then pressurized with nitrogen (15 bar). The reactor was heated to 180 °C in 1 h and this temperature was maintained for 3 h. The reactor was then cooled down in an ice bath. After releasing the gas and opening the reactor, the solvent was evaporated *in vacuo* to obtain levulinic acid as a yellow-brown oil in 97% (169 mg, 1.45 mmol) yield.

### Example 10bis: Synthesis of levulinic acid from cis,cis-muconic acid with improved separation procedure[Experiment # 61bis]

The synthesis was carried out according to Example 10, with the difference that after releasing the gas and opening the reactor, the mixture was acidified (pH < 2) with aq. HCl and continuously extracted with ethyl acetate for 24 h. The organic phase was dried over Na₂SO₄ and evaporated *in vacuo* to obtain levulinic acid as a light brown oil in quantitative (178 mg, 1.53 mmol) yield.

### Example 11: Synthesis of levulinic acid from cis,cis-muconic acid monomethyl ester [(2Z,4Z)-6-methoxy-6-oxohexa-2,4-dienoic acid] [Experiment # 62]

(2*Z*,4*Z*)-6-Methoxy-6-oxohexa-2,4-dienoic acid was synthesized according to a literature procedure, see Jastrzebski, Robin, et al., 2015.

The experiment was performed according to General Procedure C, using (2*Z*,4*Z*)-6-methoxy-6-oxohexa-2,4-dienoic acid (47 mg, 0.3 mmol) and water (3 mL) for 3 h at 180 °C under 15 bar N₂ pressure. Levulinic acid was obtained in 95% ¹H NMR yield.

### Example 12: Synthesis of levulinic acid from cis,trans-muconic acid [Experiment # 63]

*cis,trans*-Muconic acid was synthesized according to a literature procedure, see US8426639B2 (example 77).

The experiment was performed according to General Procedure C, using *cis,trans*-muconic acid (43 mg, 0.3 mmol) and water (3 mL) for 3 h at 180 °C under 15 bar N₂ pressure. Levulinic acid was obtained in 107% ¹H NMR yield. By repeating the experiment after recalibrating the temperature controller of the reactor, levulinic acid was obtained in 109% ¹H NMR yield.

### Example 13: Synthesis of levulinic acid from cis,trans-muconic acid monosodium salt [Experiment # 64]

*cis,trans*-Muconic acid was synthesized according to a literature procedure, see US8426639B2 (example 77).

The experiment was performed according to modified General Procedure C, using *cis,trans-*muconic acid (43 mg, 0.3 mmol), 1 M aq. NaOH (0.3 mL, 0.3 mmol, 1 eq.) and water (2.7 mL) for 3 h at 180 °C under 15 bar N₂ pressure. Workup consisted of the addition of internal standard, acetone and concentrated HCl to acidify the solutions. After centrifugation, about half of the mixture was evaporated, dissolved in DMSO-*d₆* and analyzed using NMR. Levulinic acid was obtained in 103% ¹H NMR yield.

### Example 14: Synthesis of levulinic acid cis,trans-muconic acid disodium salt [Experiment # 65]

The experiment was performed according to modified General Procedure C, using *cis,trans*-muconic acid (43 mg, 0.3 mmol), 1 M aq. NaOH (0.6 mL, 0.6 mmol, 2 eq.) and water (2.4 mL) for 3 h at 180 °C under 15 bar N₂ pressure. Workup consisted of the addition of internal standard, acetone and concentrated HCl to acidify the solutions. After centrifugation, about half of the mixture was evaporated, dissolved in DMSO-*d₆* and analyzed using NMR. Levulinic acid was obtained in 96% ¹H NMR yield. *cis,trans*-Muconic acid was recovered in 8% ¹H NMR yield.

### Example 15: Synthesis of levulinic acid from trans,trans-muconic acid [Experiment # 66]

The experiment was performed according to General Procedure C, using *trans,trans-*muconic acid (43 mg, 0.3 mmol) and water (3 mL) for 3 h at 180 °C under 15 bar N₂ pressure. No levulinic acid was obtained and *trans,trans*-muconic acid was recovered in 59% ¹H NMR yield.

### Example 16: Synthesis of levulinic acid from trans,trans-muconic acid [Experiment # 67]

The experiment was performed according to General Procedure C, using *trans,trans-*muconic acid (43 mg, 0.3 mmol) and water (3 mL) for 3 h at 250 °C under 50 bar N₂ pressure. Levulinic acid was obtained in 21% ¹H NMR yield. No *trans,trans*-muconic acid was observed in the ¹H NMR spectrum.

### Example 17: Acid concentration screening for the synthesis of levulinic acid from trans, trans-muconic acid

These experiments were performed according to General Procedure C, using *trans,trans*-muconic acid (43 mg, 0.3 mmol), the indicated amount of HCl and water (3 mL) for 3 h at 180 °C under 15 bar N₂ pressure. The ¹H NMR yields (dimethyl sulfone internal standard) are reported in the table below.

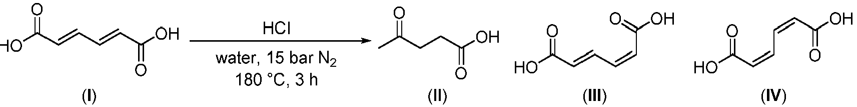

| Experiment # | eq. HCl | C_{HCl} (M) | Recovered I (%)^{a} | Yield II (%)^{a} | Yield III (%)^{a} | Yield IV (%)^{a} |
|---|---|---|---|---|---|---|
| 68 | 0.0 | 0.00 | 59 | 0 | 0 | 0 |
| 69 | 0.5 | 0.05 | 60 | 11 | 0 | 0 |
| 70 | 1.0 | 0.10 | 61 | 19 | 0 | 0 |
| 71 | 2.0 | 0.20 | 47 | 27 | 0 | 0 |
| 72 | 5.0 | 0.50 | 34 | 49 | 0 | 0 |
| 73 | 10 | 1.00 | 14 | 80 | 0 | 0 |

| | | | | | | |
|---|---|---|---|---|---|---|
| ^{a} As there is an experimental error on each individual value determined by ¹H NMR, the mass balance can be more than 100%. | | | | | | |

### Example 18: Base concentration screening for the synthesis of levulinic acid from trans, trans-muconic acid

These experiments were performed according to a modified General Procedure C, using *trans,trans*-muconic acid (43 mg, 0.3 mmol), the indicated amount of NaOH and water (3 mL) for 3 h at 180 °C under 15 bar N₂ pressure. Workup consisted of the addition of internal standard, acetone and concentrated HCl to acidify the solutions. After centrifugation, about half of the mixture was evaporated, dissolved in DMSO-*d₆*, filtered if required and analyzed using NMR. The ¹H NMR yields (dimethyl sulfone or dimethylmalonic acid internal standard) are reported in the table below.

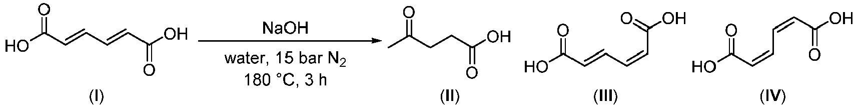

| Experiment # | eq. NaOH | C_{NaOH} (M) | Recovered I (%)^{a} | Yield II (%)^{a} | Yield III (%)^{a} | Yield IV (%)^{a} |
|---|---|---|---|---|---|---|
| 74 | 0.0 | 0.00 | 61 | 0 | 0 | 0 |
| 75 | 0.5 | 0.05 | 37 | 0 | 0 | 0 |
| 76 | 1.0 | 0.10 | 69 | 0 | 0 | 0 |
| 77 | 2.0 | 0.20 | 80 | trace | 0 | 0 |
| 78 | 5.0 | 0.50 | 4 | 46 | 9 | 1 |
| 79 | 10 | 1.00 | 1 | 57 | 11 | 1 |

| | | | | | | |
|---|---|---|---|---|---|---|
| ^{a} As there is an experimental error on each individual value determined by ¹H NMR, the mass balance can be more than 100%. | | | | | | |

### Example 19: Synthesis of muconolactone (2,5-dihydro-5-oxo-2-furanacetic acid) from cis,cis-muconic acid [Experiment # 80]

The reaction was performed using a modified literature procedure, see US20170129839A1 (example 3-1), starting from *cis,cis*-muconic acid instead of *cis,trans-*muconic acid. To an oven-dried round-bottomed flask was added cis,cis-muconic acid (355 mg, 2.5 mmol) and dry acetonitrile (25 mL) under argon. The suspension was stirred at 75 °C for 20 h. Afterwards, the solvent was evaporated and the crude product was purified using automated flash chromatography (Hept/EtOAc gradient + 2% AcOH; 12 g SiO₂ column). Muconolactone was obtained as an off-white solid in 75% yield (268 mg, 1.88 mmol). *cis,trans*-Muconic acid was isolated as a side product in 15% (52 mg, 0.37 mmol) yield.

### Example 20: Synthesis of levulinic acid from muconolactone (2,5-dihydro-5-oxo-2-furanacetic acid) [Experiment # 81]

The experiment was performed according to General Procedure C, using muconolactone (43 mg, 0.3 mmol) and water (3 mL) for 3 h at 180 °C under 15 bar N₂ pressure. Levulinic acid was obtained in 104% ¹H NMR yield. By repeating the experiment after recalibrating the temperature controller of the reactor, levulinic acid was obtained in 99%¹H NMR yield.

### Example 21: Synthesis of levulinic acid from muconolactone sodium salt (sodium 2,5-dihydro-5-oxo-2-furanacetate) [Experiment # 82]

The experiment was performed according to modified General Procedure C, using muconolactone (43 mg, 0.3 mmol), 1 M aq. NaOH (0.3 mL, 0.3 mmol, 1 eq.) and water (2.7 mL) for 3 h at 180 °C under 15 bar N₂ pressure. Workup consisted of the addition of internal standard, acetone and concentrated HCl to acidify the solutions. After centrifugation, about half of the mixture was evaporated, dissolved in DMSO-*d₆* and analyzed using NMR. Levulinic acid was obtained in 108% ¹H NMR yield.

### Example 22: Synthesis of levulinic acid from muconic acid dilactone (tetrahydrofuro[3,2-b]furan-2,5-dione) [Experiment # 83]

Muconic acid dilactone was synthesized using a literature procedure, see Hizuka, Michiyo, et al., 1988.

The experiment was performed according to General Procedure C, using muconic acid dilactone (43 mg, 0.3 mmol) and water (3 mL) for 3 h at 180 °C under 15 bar N₂ pressure. Levulinic acid was obtained in 107% ¹H NMR yield.

### Example 23: Synthesis of 3-methyl-4-oxopentanoic acid from (2Z,4E)-3-methylhexa-2,4-dienedioic acid [Experiment # 84]

(2*Z*,4*E*)-3-Methylhexa-2,4-dienedioic acid was synthesized as described in the literature, see Coupé, Florentin, et al., 2020.

The experiment was performed according to General Procedure C, using (2*Z*,4*E*)-3-methylhexa-2,4-dienedioic acid (47 mg, 0.3 mmol) and water (3 mL) for 3 h at 250 °C under 50 bar N₂ pressure. 3-Methyl-4-oxopentanoic acid was obtained in 94% ¹H NMR yield.

TLC-MS (ESI) [M-H]⁻ calcd. 129.06, found 129.2. By repeating the experiment after recalibrating the temperature controller of the reactor, 3-methyl-4-oxopentanoic acid was obtained in 91% ¹H NMR yield.

### Example 24: Synthesis of 3-methyl-4-oxopentanoic acid from (3-methyl-5-oxo-2,5-dihydrofuran-2-yl)acetic acid [Experiment # 85]

(3-Methyl-5-oxo-2,5-dihydrofuran-2-yl)acetic acid was synthesized as described in the literature, see Coupé, Florentin, et al., 2020.

The experiment was performed according to General Procedure C, using (3-methyl-5-oxo-2,5-dihydrofuran-2-yl)acetic acid (47 mg, 0.3 mmol) and water (3 mL) for 3 h at 250 °C under 50 bar N₂ pressure. 3-Methyl-4-oxopentanoic acid was obtained in 99% ¹H NMR yield. By repeating the experiment after recalibrating the temperature controller of the reactor, 3-methyl-4-oxopentanoic acid was obtained in 104% ¹H NMR yield.

### Example 25: Synthesis of 3-propyl-4-oxopentanoic acid from (3-propyl-5-oxo-2,5-dihydrofuran-2-yl)acetic acid [Experiment # 86]

(3-Propyl-5-oxo-2,5-dihydrofuran-2-yl)acetic acid was synthesized as described in the literature, see Coupé, Florentin, et al., 2020.

The experiment was performed according to General Procedure C, using (3-propyl-5-oxo-2,5-dihydrofuran-2-yl)acetic acid (55 mg, 0.3 mmol) and water (3 mL) for 3 h at 250 °C under 50 bar N₂ pressure. 3-Propyl-4-oxopentanoic acid was obtained in 97% ¹H NMR yield.

TLC-MS (ESI) [M-H]⁻ calcd. 157.09, found 157.3. By repeating the experiment after recalibrating the temperature controller of the reactor, 3-propyl-4-oxopentanoic acid was obtained in 82%¹H NMR yield.

### Example 26: Synthesis of 3-tert-butyl-4-oxopentanoic acid from (3-tert-butyl-5-oxo-2,5-dihydrofuran-2-yl)acetic acid [Experiment # 87]

(3-*tert*-butyl-5-oxo-2,5-dihydrofuran-2-yl)acetic acid was synthesized as described in the literature, see Coupé, Florentin, et al., 2020.

The experiment was performed according to General Procedure C, using (3-*tert*-butyl-5-oxo-2,5-dihydrofuran-2-yl)acetic acid (59 mg, 0.3 mmol) and water (3 mL) for 3 h at 250 °C under 50 bar N₂ pressure. 3-*tert*-Butyl-4-oxopentanoic acid was obtained in 93% ¹H NMR yield.

TLC-MS (ESI) [M-H]⁻ calcd. 171.10, found 171.3. By repeating the experiment after recalibrating the temperature controller of the reactor, 3-*tert*-butyl-4-oxopentanoic acid was obtained in 102% ¹H NMR yield.

### Example 27: Synthesis of 3-phenyl-4-oxopentanoic acid from (3-phenyl-5-oxo-2,5-dihydrofuran-2-yl)acetic acid [Experiment # 88]

(3-Phenyl-5-oxo-2,5-dihydrofuran-2-yl)acetic acid was synthesized as described in the literature, see Coupé, Florentin, et al., 2020.

The experiment was performed according to General Procedure C, using (3-phenyl-5-oxo-2,5-dihydrofuran-2-yl)acetic acid (65 mg, 0.3 mmol) and water (3 mL) for 3 h at 250 °C under 50 bar N₂ pressure. 3-Phenyl-4-oxopentanoic acid was obtained in 96% ¹H NMR yield.

TLC-MS (ESI) [M-H]⁻ calcd. 191.07, found 191.

### Example 28: Synthesis of levulinic acid from (2E)-3-[2-(carboxymethyl)-5-oxo-2,5-dihydrofuran-3-yl]prop-2-enoic acid [Experiment # 89]

(2*E*)-3-[2-(carboxymethyl)-5-oxo-2,5-dihydrofuran-3-yl]prop-2-enoic acid was synthesized as described in the literature, see Coupé, Florentin, et al., 2020.

The experiment was performed according to General Procedure C, using (2*E*)-3-[2-(carboxymethyl)-5-oxo-2,5-dihydrofuran-3-yl]prop-2-enoic acid (64 mg, 0.3 mmol) and water (3 mL) for 3 h at 250 °C under 50 bar N₂ pressure. Levulinic acid was obtained in 49% ¹H NMR yield.

### Example 29: Synthesis of levulinic acid from [3-(methoxycarbonyl)-5-oxo-2,5-dihydrofuran-2-yl]acetic acid [Experiment # 90]

[3-(Methoxycarbonyl)-5-oxo-2,5-dihydrofuran-2-yl]acetic acid was synthesized as described in the literature, see Coupé, Florentin, et al., 2020.

The experiment was performed according to General Procedure C, using [3-(methoxycarbonyl)-5-oxo-2,5-dihydrofuran-2-yl]acetic acid (60 mg, 0.3 mmol) and water (3 mL) for 3 h at 250 °C under 50 bar N₂ pressure. Levulinic acid was obtained in 104% ¹H NMR yield.

### Example 30: Temperature screening for the synthesis of methyl levulinate from cis,cis-muconic acid

These experiments were performed according to General Procedure A, using *cis,cis-*muconic acid (43 mg, 0.3 mmol) and methanol (3 mL) for 3 h under 90 bar of N₂ pressure. The ¹H NMR yields (1,3,5-trimethoxybenzene internal standard) are reported in the table below.

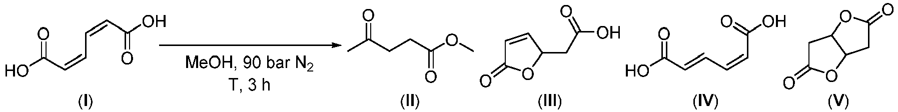

| Experiment # | T (°C) | Recovered I (%)^{a} | Yield II (%)^{a} | Yield III (%)^{a} | Yield IV (%)^{a} | Yield V (%)^{a} |
|---|---|---|---|---|---|---|
| 91 | 180 | 0 | 8 | 42 | 28 | 4 |
| 92 | 190 | 0 | 17 | 45 | 12 | 3 |
| 93 | 200 | 0 | 22 | 36 | 8 | 2 |
| 94 | 210 | 0 | 42 | 15 | 3 | 0 |
| 95 | 220 | 0 | 57 | 7 | 0 | 0 |
| 96 | 230 | 0 | 69 | trace | trace | trace |
| 97 | 240 | 0 | 59 | 0 | 0 | 0 |

| | | | | | | |
|---|---|---|---|---|---|---|
| ^{a} As there is an experimental error on each individual value determined by ¹H NMR, the mass balance can be more than 100%. | | | | | | |

### Example 31: Additive screening for the synthesis of methyl levulinate from cis,cis-muconic acid

These experiments were performed according to General Procedure C, using *cis,cis-*muconic acid (43 mg, 0.3 mmol), the indicated additive (60 µmol, 0.2 eq.) and methanol (3 mL) for 3 h at 180 °C under 90 bar N₂ pressure. The ¹H NMR yields (1,3,5-trimethoxybenzene internal standard) are reported in the table below.

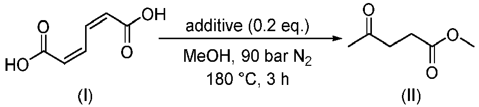

| Experiment # | Additive | Yield II (%)^{a} |
|---|---|---|
| 98 | none | 39 |
| 99 | NaOMe | 75 |
| 100 | NaOAc | 76 |
| 101 | NH₄OAc | 62 |
| 102 | tetrabutylammonium benzoate | 79 |
| 103 | Al(OH)₃ | 65 |
| 104 | neutral alumina | 46 |
| 105 | basic alumina | 61 |
| 106 | silica | 58 |
| 107 | NH₃ | 65 |
| 108 | NEt₃ | 69 |
| 109 | EtNiPr₂ | 82 |
| 110 | piperidine | 60 |
| 111 | 2,2,6,6-tetramethylpiperidine | 80 |
| 112 | 1-azabicyclo[2.2.2]octane | 80 |
| 113 | 1,4-diazabicyclo[2.2.2]octane | 80 |
| 114 | 1,8-bis(dimethylamino)naphthalene | 84 |
| 115 | 2-hydroxyethylamine | 61 |
| 116 | pyridine | 67 |
| 117 | 4-dimethylaminopyridine | 74 |
| 118 | 2,6-dimethylpyridine | 68 |
| 119 | *N*-methylimidazole | 77 |
| 120 | 1,8-diazabicyclo[5.4.0]undec-7-ene | 76 |
| 121 | *N,N*-dimethylaniline | 65 |
| 122 | 1,1,3,3-tetramethylguanidine | 69 |
| 123 | L-arginine | 61 |
| 124 | L-histidine | 66 |
| 125 | L-lysine | 56 |
| 126 | MgCl₂ | 58 |
| 127 | SnCl₄ | 4 |
| 128 | H₂SO₄ (0.25 eq.) | 23 |
| 129 | AcOH (1 eq.) | 46 |
| 130 | TsOH.H₂O (0.5 eq.) | 28 |

| | | |
|---|---|---|
| ^{a} As there is an experimental error on each individual value determined by ¹H NMR, the mass balance can be more than 100%. | | |

### Example 32: One-pot conversion of catechol to methyl levulinate [Experiment # 131]

The oxidation of catechol under air was executed according to a modified literature procedure, see Jastrzebski, Robin, et al., 2015. A glass liner equipped with a magnetic stirring bar was charged with a mixture of catechol (1.101 g, 10.0 mmol), Fe(NO₃)₃.9H₂O (20 mg, 50 µmol, 0.5 mol%), tris(2-pyridylmethyl)amine (15 mg, 20 µmol, 0.5 mol%) and 1,4-diazabicyclo[2.2.2]octane (112 mg, 1.0 mmol, 10 mol%) in methanol (100 mL). The liner was placed in the 4651 Parr reactor. The reactor was closed properly and flushed with synthetic air (10 bar), then pressurized with synthetic air (25 bar), all under vigorous stirring. The reactor was heated to 80 °C in 30 min and held at that temperature for 4 h. The heating was stopped and the reactor cooled in an ice bath for approx. 45 min. The reactor was then vented, flushed with nitrogen (3x 30 bar) and pressurized with nitrogen (85 bar). It was heated to 180 °C in 1 h and held at that temperature for 3 h. The heating was then stopped and the reactor cooled overnight. The reaction mixture was evaporated to a volume of less than 50 mL, and then partitioned between 100 mL EtOAc and 100 mL 1 M aq. HCl. Phases were separated and the aqueous layer washed again with 2x 100 mL EtOAc. The combined organic layers were washed with sat. aq. NaHCOs (50 mL), dried over MgSO₄, concentrated and purified using automated flash chromatography (40 g SiO₂ column, Hept/EtOAc gradient) to obtain methyl levulinate as a yellow oil in 16% (210 mg, 1.61 mmol) yield.

### Example 33: Synthesis of 2-methyl-4-oxopentanoic acid from (2Z,4Z)-2-methylhexa-2,4-dienedioic acid [Experiment # 132]

(2*Z*,4*Z*)-2-Methylhexa-2,4-dienedioic acid was synthesized as described in the literature, see Coupé, Florentin, et al., 2020.

The experiment was performed according to General Procedure C, using (2*Z*,4*Z*)-2-methylhexa-2,4-dienedioic acid (47 mg, 0.3 mmol) and water (3 mL) for 3 h at 250 °C under 50 bar N₂ pressure. 2-Methyl-4-oxopentanoic acid was obtained in 60% ¹H NMR yield. 4-Oxohexanoic acid was obtained as a side product in 14% ¹H NMR yield. TLC-MS (ESI) [M-H]⁻ calcd. 129.06, found 129.

### Example 34: Solvent and additive screening for the synthesis of methyl levulinate from cis,cis-muconic acid

These experiments were performed according to General Procedure C, using *cis,cis-*muconic acid (43 mg, 0.3 mmol), the indicated additive (60 µmol, 0.2 eq.) and alkyl alcohol (3 mL) for 3 h at 180 °C under 80 bar N₂ pressure. The ¹H NMR yields (1,3,5-trimethoxybenzene internal standard) are reported in the table below.

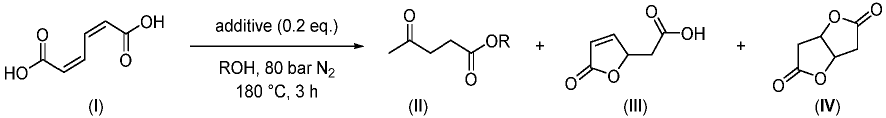

| Experiment # | R | Additive | Yield II (%)^{a} | Yield III (%)^{a} | Yield IV (%)^{a} |
|---|---|---|---|---|---|
| 133 | Et | none | 34 | 34 | 2 |
| 134 | Et | EtNiPr₂ | 78 | 0 | 0 |
| 135 | Et | 1-azabicyclo[2.2.2]octane | 83 | 0 | 0 |
| 136 | Et | 2,2,6,6-tetramethylpiperidine | 79 | 0 | 0 |
| 137 | iPr | 1,4-diazabicyclo[2.2.2]octane | 57 | 0 | 0 |
| 138 | *n*-Bu | EtNiPr₂ | 75 | 0 | 0 |
| 139 | *n*-Bu | 1,4-diazabicyclo[2.2.2]octane | 85 | 0 | 0 |
| 140 | *n*-Bu | 1-azabicyclo[2.2.2]octane | 87 | 0 | 0 |
| 141 | *n*-Bu | 2,2,6,6-tetramethylpiperidine | 79 | 0 | 0 |

### REFERENCES

1. Coupé, Florentin, et al. "Sustainable oxidative cleavage of catechols for the synthesis of muconic acid and muconolactones including lignin upgrading." Green Chemistry 22.18 (2020): 6204-6211.
2. US8426639B2, "Preparation of trans, trans muconic acid and trans, trans muconates", example 77.
3. US20170129839A1 "ISOMERIZATION OF MUCONIC ACID", example 3-1.
4. Hizuka, Michiyo, et al., "Preparation of 2, 6-Dioxabicyclo [3.3.0] octan-3,7-dione and Its Application to the Synthesis of (±)-Eldanolide." Chemical and pharmaceutical bulletin 36.4 (1988): 1550-1553.
**5.** Jastrzebski, Robin, et al. "Sustainable production of dimethyl adipate by non-heme iron (III) catalysed oxidative cleavage of catechol." Catalysis Science & Technology 5.4 (2015): 2103-2109.

## Claims

1. A process for preparing a levulinic acid, levulinate ester or salt thereof, comprising the steps of:
a) providing a reaction mixture comprising:
- a polar protic solvent; and
- a muconic compound selected from the list comprising: muconic acid, muconate salt, muconate ester, mucono(di)lactone, and represented by general formula (I): 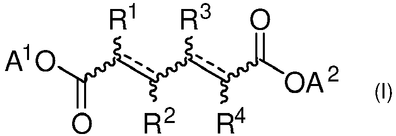 wherein for general formula (I):
- A¹, A² are independently selected from the list comprising: H, alkyl, and a cation;
- R¹, R², R³, R⁴ are independently selected form the list comprising H, alkyl, alkenyl, carboxyl, aryl, and at least one of R², R³ is H;
- R¹C-CR², and R³C-CR⁴ are double C-C bonds;
thereby constituting group formula (IA) 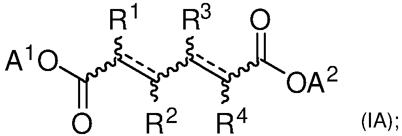 or
- A¹ represents a direct bond connecting the oxygen to which it is attached to the carbon atom at the R³ position thereby forming a 5 membered ring;
- A² is selected from the list comprising: H, alkyl, and cation;
- R¹, R², R⁴ are independently selected from the list comprising: H, alkyl, alkenyl, carboxyl, aryl;
- R¹C-CR² is a double C-C bond, and R³C-CR⁴ is a single C-C bond;
thereby constituting formula group (IB) 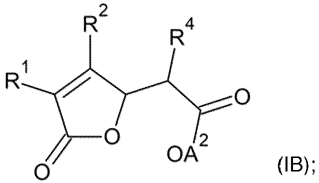 or
- A¹, respectively A² each represent respectively a direct bond connecting the oxygen to which it is attached to the carbon atom at the R³ position, respectively R² thereby forming a fused system of two 5-membered rings;
- R¹, R⁴ are independently selected from the list comprising: H, alkyl, alkenyl, carboxyl, aryl;
- R¹C-CR², and R³C-CR⁴ are single C-C bonds;
thereby constituting formula group (IC) 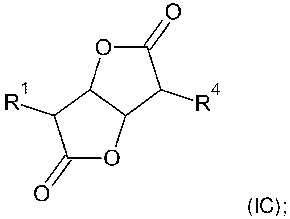 and
b) heating the reaction mixture provided at step a) at a temperature Tᵢ of at least about 120°C and at a pressure Pᵢ ≥ autogenic pressure, thereby preparing the levulinic acid, or salt thereof or levulinate ester;
wherein the levulinic acid, or salt thereof or levulinate ester is represented by formula (II): 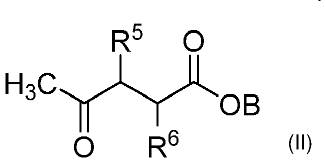 wherein
- B selected from the group comprising: H, alkyl, inorganic cation, organic cation; and
- R⁵, R⁶ are selected from, and not limited to, the group comprising: H, alkyl, alkenyl, carboxyl, aryl.

2. The process according to any one of the previous claims, wherein step b) comprises heating the reaction mixture at a temperature Tᵢ of at least 160°C, preferably at least 180°C.

3. The process according to any one of the previous claims, wherein at step a) the polar protic solvent is water.

4. The process according to claim 3, wherein at step a) water is not provided to contain any basic or acidic additives, so that the reaction mixture is not provided with an adjusted pH, which differs from the pH of the reaction mixture provided by the muconic compound or compounds alone.

5. The process according to claim 3 or 4, wherein at step a) the reaction mixture consists of water and one or more muconic compounds.

6. The process according to claim 1 or 2, wherein the polar protic solvent is an alkyl alcohol, preferably selected form the list comprising: methanol, ethanol, isopropanol, propanol and butanol.

7. The process according to claim 6, wherein at step a) the polar protic solvent is methanol.

8. The process according to claim 6 or 7, wherein the reaction mixture further comprises a basic catalyst.

9. The process according to claim 8, wherein the basic catalyst comprises one or more sp3 hybridized nitrogen atoms.

10. The process according to claim 8 or 9, wherein the basic catalyst is selected from the list comprising: diisopropylethylamine, 2,2,6,6-tetramethylpiperidine, 1-azabicyclo[2.2.2]octane, 1,4-diazabicyclo[2.2.2]octane, 1,8-bis(dimethylamino)naphthalene.

11. The process according to any one of the previous claims, wherein the muconic compound is of group formula (IA).

12. The process according to any one of the previous claims, wherein step a) comprises providing the muconic compound in a cis,cis-, cis,trans- or trans,cis-configuration, preferably a cis,cis-configuration.

13. The process according to any one of the previous claims, wherein step b) comprises heating the reaction mixture in a non-oxidizing atmosphere.

14. The process according to any one of the previous claims, wherein steps a) and b) are performed subsequently, without isolation of any intermediates.

## Patentansprüche

1. Verfahren zum Herstellen einer Lävulinsäure, eines Lävulinatesters oder eines Salzes davon, umfassend die Schritte:
a) Bereitstellen eines Reaktionsgemisches, umfassend:
- ein polares protisches Lösungsmittel; und
- eine Muconverbindung, die aus der Liste ausgewählt ist, umfassend: Muconsäure, Muconatsalz, Muconatester, Mucono(di)lacton, und dargestellt durch eine allgemeine Formel (I): 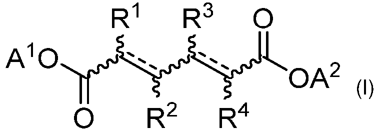 wobei für die allgemeine Formel (I):
- A¹, A² unabhängig voneinander aus der Liste ausgewählt sind, umfassend: H, Alkyl und ein Kation;
- R¹, R², R³, R⁴ unabhängig voneinander aus der Liste ausgewählt sind, umfassend H, Alkyl, Alkenyl, Carboxyl, Aryl und mindestens eines von R², R³ H ist;
- R¹C-CR² und R³C-CR⁴ doppelte C-C-Bindungen sind;
wobei dadurch Gruppenformel (IA) gebildet wird 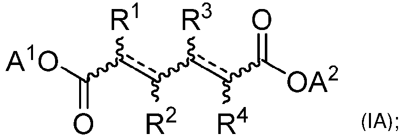 oder
- A¹ eine direkte Bindung darstellt, die den Sauerstoff, an den es gebunden ist, mit dem Kohlenstoffatom an der R³-Position verbindet, wobei dadurch ein 5-gliedriger Ring gebildet wird;
- A² aus der Liste ausgewählt ist, umfassend: H, Alkyl und Kation;
- R¹, R², R⁴ unabhängig voneinander aus der Liste ausgewählt sind, umfassend: H, Alkyl, Alkenyl, Carboxyl, Aryl;
- R¹C-CR² eine doppelte C-C-Bindung ist und R³C-CR⁴ eine einzelne C-C-Bindung ist;
wobei dadurch Formelgruppe (IB) gebildet wird 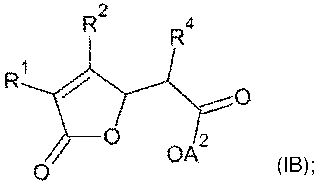 oder
- A¹ beziehungsweise A² jedes jeweils eine direkte Bindung darstellen, die den Sauerstoff an den es gebunden ist, mit dem Kohlenstoffatom an der R³-Position, beziehungsweise R² verbindet, wobei dadurch ein anneliertes System aus zwei 5-gliedrigen Ringen gebildet wird;
- R¹, R⁴ unabhängig voneinander aus der Liste ausgewählt sind, umfassend: H, Alkyl, Alkenyl, Carboxyl, Aryl;
- R¹C-CR² und R³C-CR⁴ einzelne C-C-Bindungen sind;
wobei dadurch Formelgruppe (IC) gebildet wird 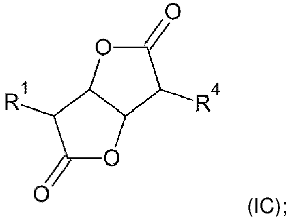 und
b) Erhitzen des Reaktionsgemisches, das in Schritt a) bereitgestellt wird, bei einer Temperatur Tᵢ von mindestens etwa 120 °C und bei einem Druck Pᵢ ≥ autogenem Druck, wobei dadurch die Lävulinsäure oder das Salz davon oder der Lävulinsäureester hergestellt wird;
wobei die Lävulinsäure oder das Salz davon oder der Lävulinsäureester dargestellt wird durch die Formel (II): 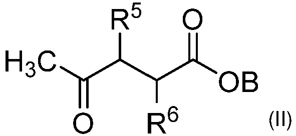 wobei
- B aus der Gruppe ausgewählt ist, umfassend: H, Alkyl, anorganisches Kation, organisches Kation; und
- R⁵, R⁶ aus der Gruppe ausgewählt sind, und nicht beschränkt sind auf die Gruppe, umfassend: H, Alkyl, Alkenyl, Carboxyl, Aryl.

2. Verfahren nach einem der vorhergehenden Ansprüche, wobei Schritt b) das Erhitzen des Reaktionsgemisches bei einer Temperatur Tᵢ von mindestens 160 °C, vorzugsweise mindestens 180 °C, umfasst.

3. Verfahren nach einem der vorhergehenden Ansprüche, wobei in Schritt a) das polare protische Lösungsmittel Wasser ist.

4. Verfahren nach Anspruch 3, wobei in Schritt a) kein Wasser bereitgestellt wird, das beliebige basische oder saure Zusätze enthält, sodass das Reaktionsgemisch mit keinem eingestellten pH-Wert versehen ist, der sich von dem pH-Wert des Reaktionsgemisches, der durch die Muconverbindung(en) allein bereitgestellt wird, unterscheidet.

5. Verfahren nach Anspruch 3 oder 4, wobei in Schritt a) das Reaktionsgemisch aus Wasser und einer oder mehreren Muconverbindungen besteht.

6. Verfahren nach Anspruch 1 oder 2, wobei das polare protische Lösungsmittel ein Alkylalkohol ist, der vorzugsweise aus der Liste ausgewählt ist, umfassend: Methanol, Ethanol, Isopropanol, Propanol und Butanol.

7. Verfahren nach Anspruch 6, wobei in Schritt a) das polare protische Lösungsmittel Methanol ist.

8. Verfahren nach Anspruch 6 oder 7, wobei das Reaktionsgemisch ferner einen basischen Katalysator umfasst.

9. Verfahren nach Anspruch 8, wobei der basische Katalysator ein oder mehrere sp3-hybridisierte Stickstoffatome umfasst.

10. Verfahren nach Anspruch 8 oder 9, wobei der basische Katalysator aus der Liste ausgewählt ist, umfassend: Diisopropylethylamin, 2,2,6,6-Tetramethylpiperidin, 1-Azabicyclo[2.2.2]octan, 1,4-Diazabicyclo[2.2.2]octan, 1,8-Bis(dimethylamino)naphthalin.

11. Verfahren nach einem der vorhergehenden Ansprüche, wobei die Muconverbindung von Gruppenformel (IA) ist.

12. Verfahren nach einem der vorhergehenden Ansprüche, wobei Schritt a) das Bereitstellen der Muconverbindung in einer cis,cis-, cis,trans- oder trans,cis-Konfiguration, vorzugsweise einer cis,cis-Konfiguration, umfasst.

13. Verfahren nach einem der vorhergehenden Ansprüche, wobei Schritt b) das Erhitzen des Reaktionsgemisches in einer nichtoxidierenden Atmosphäre umfasst.

14. Verfahren nach einem der vorhergehenden Ansprüche, wobei die Schritte a) und b) nacheinander ohne Isolierung beliebiger Intermediate durchgeführt werden.

## Revendications

1. Procédé permettant de préparer un acide lévulinique, un ester de lévulinate ou un sel de celui-ci, comprenant les étapes consistant à :
a) fournir un mélange réactionnel comprenant :
- un solvant protique polaire ; et
- un composé muconique choisi dans la liste comprenant : acide muconique, sel de muconate, ester de muconate, mucono(di)lactone, et représenté par la formule générale (I) : 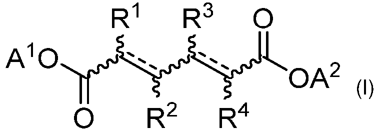 dans lequel pour la formule générale (I) :
- A¹, A² sont indépendamment choisis dans la liste comprenant : H, alkyle et un cation ;
- R¹, R², R³, R⁴ sont indépendamment choisis dans la liste comprenant H, alkyle, alcényle, carboxyle, aryle, et au moins l'un parmi R², R³ est H ;
- R¹C-CR² et R³C-CR⁴ sont des doubles liaisons C-C ;
constituant de ce fait une formule de groupe (IA) 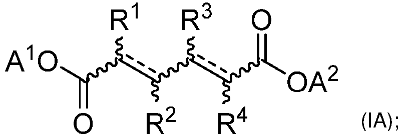 ou
- A¹ représente une liaison directe reliant l'oxygène auquel il est fixé à l'atome de carbone à la position de R³ en formant de ce fait un cycle à 5 chaînons ;
- A² est choisi parmi la liste comprenant : H, alkyle et cation ;
- R¹, R², R⁴ sont indépendamment choisis dans la liste comprenant : H, alkyle, alcényle, carboxyle, aryle ;
- R¹C-CR² est une double liaison C-C, et R³C-CR⁴ est une liaison simple C-C ;
constituant de ce fait une formule de groupe (IB) 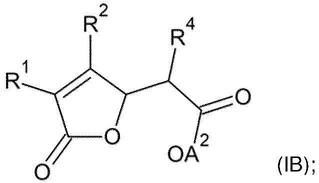 ou
- A¹, ou respectivement A² représentent chacun respectivement une liaison directe reliant l'oxygène auquel il est fixé à l'atome de carbone à la position de R³, ou respectivement R² en formant de ce fait un système condensé de deux cycles à 5 chaînons ;
- R¹, R⁴ sont indépendamment choisis dans la liste comprenant : H, alkyle, alcényle, carboxyle, aryle ;
- R¹C-CR² et R³C-CR⁴ sont des liaisons simples C-C ;
constituant de ce fait une formule de groupe (IC) 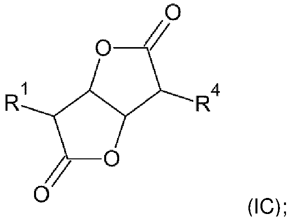 et
b) chauffer le mélange réactionnel fourni à l'étape a) à une température Tᵢ d'au moins environ 120 °C et à une pression Pᵢ ≥ pression autogène, en préparant de ce fait l'acide lévulinique, ou un sel de celui-ci ou un ester de lévulinate ;
dans lequel l'acide lévulinique, ou un sel de celui-ci ou l'ester de lévulinate est représenté par la formule (II) : 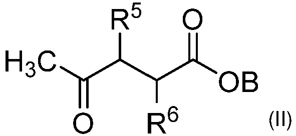 dans lequel
- B choisi dans le groupe comprenant : H, alkyle, cation inorganique, cation organique ; et
- R⁵, R⁶ sont choisis parmi, et sans s'y limiter, le groupe comprenant : H, alkyle, alcényle, carboxyle, aryle.

2. Procédé selon l'une quelconque des revendications précédentes, dans lequel l'étape b) comprend le chauffage du mélange réactionnel à une température Tᵢ d'au moins 160 °C, de préférence au moins 180 °C.

3. Procédé selon l'une quelconque des revendications précédentes, dans lequel à l'étape a) le solvant protique polaire est de l'eau.

4. Procédé selon la revendication 3, dans lequel à l'étape a) l'eau fournie ne contient pas de quelconques additifs basiques ou acides, de sorte que le mélange réactionnel n'est pas fourni avec un pH ajusté, qui diffère du pH du mélange réactionnel fourni par le composé ou les composés muconiques seuls.

5. Procédé selon la revendication 3 ou 4, dans lequel à l'étape a) le mélange réactionnel est constitué d'eau et d'un ou plusieurs composés muconiques.

6. Procédé selon la revendication 1 ou 2, dans lequel le solvant protique polaire est un alcool alkylique, de préférence choisi dans la liste comprenant : méthanol, éthanol, isopropanol, propanol et butanol.

7. Procédé selon la revendication 6, dans lequel à l'étape a) le solvant protique polaire est du méthanol.

8. Procédé selon la revendication 6 ou 7, dans lequel le mélange réactionnel comprend en outre un catalyseur basique.

9. Procédé selon la revendication 8, dans lequel le catalyseur basique comprend un ou plusieurs atomes d'azote à hybridation sp3.

10. Procédé selon la revendication 8 ou 9, dans lequel le catalyseur basique est choisi dans la liste comprenant : di-isopropyléthylamine, 2,2,6,6-tétraméthylpipéridine, 1-azabicyclo[2.2.2]octane, 1,4-diazabicyclo[2.2.2]octane, 1,8-bis(diméthylamino)naphtalène.

11. Procédé selon l'une quelconque des revendications précédentes, dans lequel le composé muconique est de formule de groupe (IA).

12. Procédé selon l'une quelconque des revendications précédentes, dans lequel l'étape a) comprend la fourniture du composé muconique dans une configuration cis,cis, cis,trans ou trans,cis, de préférence une configuration cis,cis.

13. Procédé selon l'une quelconque des revendications précédentes, dans lequel l'étape b) comprend le chauffage du mélange réactionnel dans une atmosphère non oxydante.

14. Procédé selon l'une quelconque des revendications précédentes, dans lequel les étapes a) et b) sont mises en œuvre ultérieurement, sans isolement de quelconques intermédiaires.
